# EUROPEAN PATENT SPECIFICATION

(11) **EP 3 388 018 B1**
(45) Date of publication and mention of the grant of the patent: **09.09.2020**
(21) Application number: 16857856.5
(22) Date of filing: 24.10.2016
(51) Int. Cl.: A61C 7/08, A61C 7/36

(54) **MANDIBLE TEETH FIXING ORAL DEVICE**
ORALE FIXATIONSVORRICHTUNG FÜR UNTERKIEFERZÄHNE
DISPOSITIF BUCCAL DE FIXATION DE DENTS DE MANDIBULE

(30) Priority: 22.10.2015 KR 20150147532
(43) Date of publication of application: 17.10.2018
(73) Proprietor: Choi, Hyun Jin, Incheon 22003 (KR)
(72) Inventor: PARK, Young Hyon, Incheon 22003 (KR); PARK, Jun Young, Incheon 22003 (KR); PARK, Jun Won, Incheon 22003 (KR); CHOI, Hyun Jin, Incheon 22003 (KR)
(74) Representative: Brann AB
(86) International application number: PCT/KR2016/011987
(87) International publication number: WO 2017/069599

(56) References cited:
- EP-A1- 2 491 901
- WO-A1-2014/096341
- KR-B1- 101 133 537
- KR-B1- 101 463 021
- KR-B1- 101 505 773
- KR-B1- 101 542 349
- US-A1- 2003 224 312
- US-A1- 2003 225 594
- US-A1- 2005 037 311

## Description

### TECHNICAL FIELD

The present invention relates to an oral appliance, and more particularly, to an oral appliance fixed to lower jaw in which a fixing part to teeth is provided in a lower jaw.

### BACKGROUND ART

During sleep, a person's tongue often relaxes backward within an oral cavity. This relaxation can restrict the person's airway, causing the airway to become narrowed.

In this state, inhaled air may not entirely enter the narrowed airway and instead remain in the oral cavity. Also, residual air vibrates soft tissues around the airway to generate noise, which is commonly known as snoring.

Snoring may be frequently accompanied by obstructive sleep apnea.

In order to prevent snoring and sleep apnea, a CPAP (Constant Positive Airway Press device), which forcibly supplies air to the airway during sleep, may be used. However, while such a CPAP may be effective for snoring and sleep apnea, many people find it to be inconvenient to use and costly, and the CPAP sometimes leads to certain side effects.

Also, a surgical intervention to remove drooped soft tissues around such as palatopharynx, pharynx and soft palate may be considered, but this is not favorably accepted because recurrence is common and side effects can be expected.

Other solutions include the use of an oral appliance that is installed in teeth arrangements of both the upper and lower jaws within an oral cavity to prevent snoring and sleep apnea. These oral appliances force the lower jaw to be maintained forward, causing the tongue, which is attached to the lower jaw, to be forwardly pulled out, thereby relieving pressure applied to the airway by the tongue and widening the airway to allow air to flow smoothly.

These oral appliances come in two varieties: an integrated type oral appliance and an separated type oral appliance.

The integrated type oral appliance is formed by integrating an upper jaw teeth insertion part into which teeth included in a teeth arrangement of an upper jaw are inserted and a lower jaw teeth insertion part into which teeth included in a teeth arrangement of a lower jaw are inserted.

In contrast, the separated type oral appliance is formed by separating the upper jaw teeth insertion part and the lower jaw teeth insertion part.

The integrated type oral appliance has the upper jaw teeth insertion part and lower jaw teeth insertion part integrated, as mentioned above.

Thus, when the integrated type oral appliance is used, the lower jaw cannot move during sleeping after the oral appliance is inserted, causing the lower jaw to move forward by a predetermined distance. This can cause the TMJ (Temporo-Mandibular Joint), neighboring muscles or a ligaments to be stiffened or for pain to be present.

In addition, in the case of the separated type oral appliance, the upper jaw teeth insertion part and the lower jaw teeth insertion part are separated to allow the lower jaw to move during sleep, as mentioned above. Also, the upper jaw teeth insertion part and the lower jaw teeth insertion part are connected by a connection member to allow the lower jaw to move.

However, even with the separated type oral appliance, a movement of the lower jaw is restrained by the connection member that is affixed to the lower jaw, and the aforementioned problem remains unsolved.

Meanwhile, the present inventor has obtained a patent as Korean Patent No. 10-1463021 for an oral appliance fixed to upper jaw teeth.

However, according to the oral state and teeth arrangement of a patient, for example, due to loss of molars of upper jaw teeth, an oral appliance might not be fastened to the upper jaw teeth.

In this case, if a portion to which an oral appliance is fastened is simply changed from upper jaw teeth (molar) to lower jaw teeth (molar), it results in a lot of inconvenience and pain for the patient.

US 2003/225594 A1 discloses an oral appliance according to the preamble of claim 1.

Thus, there is a strong need for an oral appliance which prevents the stiffness of a temporomandibular joint and related muscles and enables a forward movement of the lower jaw teeth by allowing the movement of the lower jaw teeth while fixed to the lower jaw teeth.

### DISCLOSURE OF INVENTION

### Technical Problem

The present invention has been made in response to the demand as above, and an object of the present invention is to provide an oral appliance fixed to lower teeth that may allow the lower jaw smoothly to move even when being worn.

Further, another object of the present invention is to provide an oral appliance fixed to low teeth, which may relax the stiffness of the related muscles and ligaments stiffness caused by a forward movement of the lower jaw by permitting a movement of the low jaw.

Furthermore, still another object of the present invention is to provide an oral appliance fixed to lower teeth that may prevent the occurrence of a pain, a malocclusion or a jaw joint disorder even when worn.

### Solution to Problem

In order to achieve the above-mentioned object, an oral appliance is provided, as is out in the appended claims.

The oral appliance may further comprise a tongue tip mounting part formed on a portion of the body to allow a user's tongue to be placed thereon.

A boundary between the lower molar fixing recess and the lower incisor recess may be a canine.

The upper teeth guide part may be formed such that an extended length of an upper molar region is larger than an extended length of an upper incisor region.

The upper teeth guide part may be formed to extend from the body to a space between a cheek and an upper jaw teeth of a user to face an outer side of the upper jaw teeth.

The upper teeth guide part may be formed to extend toward a palate of a user to face an inner side of the upper jaw teeth.

The upper teeth guide part may comprise a first upper teeth guide part formed to extend from the body to a space between a cheek and an upper jaw teeth of a user to face an outer side of the upper jaw teeth and a second upper teeth guide part formed to extend from the body toward a palate of the user to face an inner side of the upper jaw teeth.

The body may be made of a medical resin or other human-safe material.

The lower incisor recess is formed to be spaced apart from lower incisors, and thus the lower incisor recess does not contact to the lower incisors.

The upper teeth recess is formed to be spaced apart from upper jaw teeth, and thus the upper teeth recess does not strongly engage with the upper jaw teeth.

The upper teeth recess and the lower teeth recess are formed such that a lower jaw teeth of a user move forward further than before wearing the oral appliance fixed to the lower jaw teeth.

The lower teeth recess may be formed such that a depth of the lower teeth recess is not larger than a height of an exposed surface of the lower jaw teeth.

### Advantageous Effects of Invention

As described above, according to an embodiment of the present invention, an upper teeth recess and a lower teeth recess in which teeth are inserted provides a gap to be spaced apart from the teeth in the vertical direction and the horizontal direction so that the lower jaw may move smoothly even when an oral appliance is worn.

Further, according to an embodiment of the present invention, the related muscles and ligaments stiffness caused by forward movement of the low jaw may be relaxed by permitting movement of the lower jaw.

Furthermore, according to an embodiment of the present invention, a pain, a malocclusion or a jaw joint disorder may not occur even when an oral appliance is worn in contrast to conventional oral appliances.

Moreover, according to an embodiment of the present invention, since air flow through an airway is facilitated, snoring, sleep apnea, rhinitis, or rhinosinusitis may be mitigated.

### BRIEF DESCRIPTION OF DRAWINGS

Fig. 1 includes perspective views of an oral appliance according to a first exemplary embodiment of the present disclosure, in which (a) is a top perspective view and (b) is a bottom perspective view;
Fig. 2 is a cross-sectional view taken along line A-A' in (a) of Fig. 1;
Fig. 3 is a view illustrating the oral appliance worn in an oral cavity according to the first exemplary embodiment of the present disclosure, in which (a) is a front view and (b) is a side view;
Fig. 4 is a cross-sectional view and a partially enlarged cross-sectional view of (b) of Fig. 3;
Fig. 5 is a cross-sectional view and a partially enlarged cross-sectional view illustrating a usage state of the oral appliance according to the first exemplary embodiment of the present disclosure;
Fig. 6 is a cross-sectional view and a partially enlarged cross-sectional view illustrating a wearing state of the oral appliance according to a second exemplary embodiment of the present disclosure;
Fig. 7 is a cross-sectional view and a partially enlarged cross-sectional view illustrating a wearing state of the oral appliance according to a third exemplary embodiment of the present disclosure;
Fig. 8 is a perspective cross-sectional view illustrating an oral appliance according to a fourth exemplary embodiment of the present disclosure;
Fig. 9a is a view illustrating an embodiment regarding an oral appliance fixed to lower jaw teeth,
Fig. 9b is a side view illustrating the oral appliance fixed to lower jaw teeth according to the embodiment illustrated in Fig. 9a,
Fig. 10 is a rear view illustrating a bottom surface of the oral appliance fixed to lower jaw teeth according to the embodiment illustrated in Fig. 9a,
Fig. 11 is a rear view illustrating an upper surface of the oral appliance fixed to lower jaw teeth according to the embodiment illustrated in Fig. 9a,
Figs. 12 to 14 are cross-sectional views illustrating a molar portion of the oral appliance fixed to lower jaw teeth according to the embodiment illustrated in Fig. 9a,
Fig. 15 is a view from the front right, illustrating an upper surface of the oral appliance fixed to lower jaw teeth according to the embodiment illustrated in Fig. 9a, and
Fig. 16 is a view illustrating the relationship among a tongue, teeth, the oral appliance fixed to lower jaw teeth, and a cross-section of an incisor portion of the oral appliance fixed to lower jaw teeth in a cross-section illustrated in Fig. 15.

### MODE FOR THE INVENTION

To help understand the foregoing features of the present invention, an oral appliance related to an exemplary embodiment of the present invention will be described in detail.

Exemplary embodiments of the present invention will now be described in detail with reference to the accompanying drawings.

The invention may, however, be exemplified in many different forms and should not be construed as being limited to the specific embodiments set forth herein. Rather, these embodiments are provided so that this invention will be thorough and complete, and will fully convey the scope of the invention to those skilled in the art.

In the drawings, the shapes and dimensions of elements may be exaggerated for clarity, and the same reference numerals will be used throughout to designate the same or like elements.

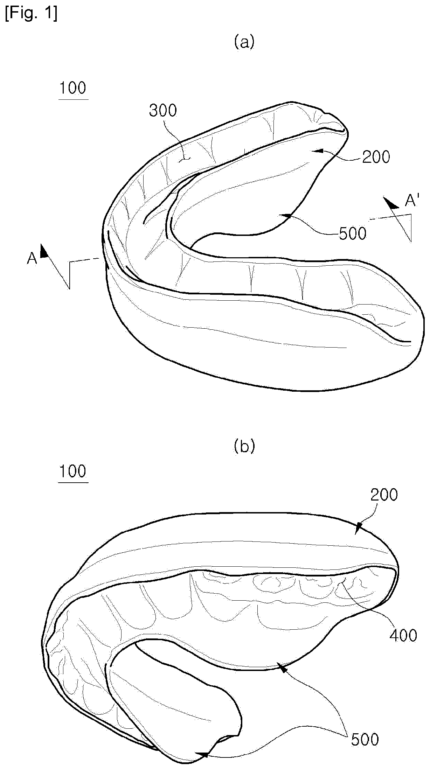

Embodiments of an upper teeth-fixed oral appliance are described with reference to Figs. 1 to 8.

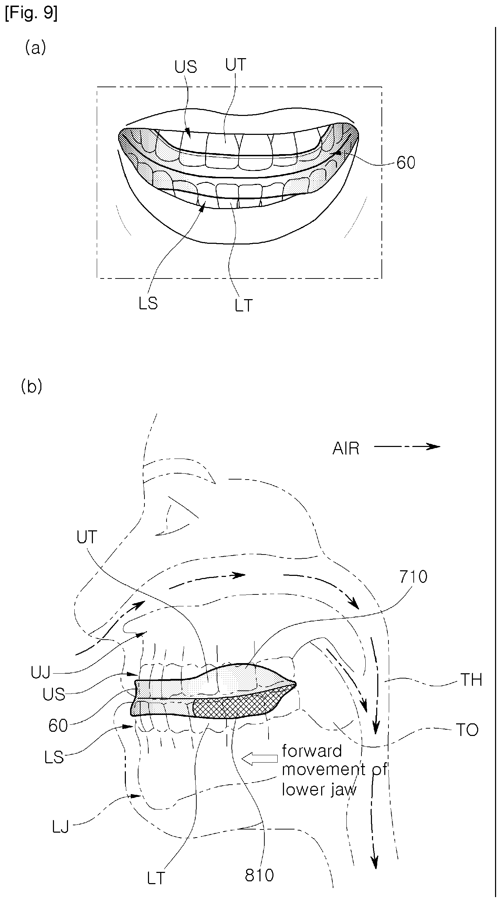

Embodiments of a lower teeth-fixed oral appliance are described with reference to Figs. 9a to 14.

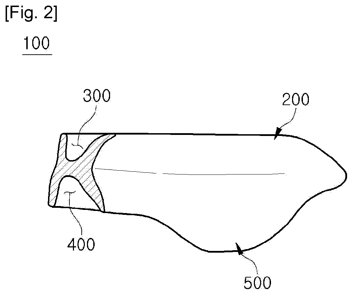

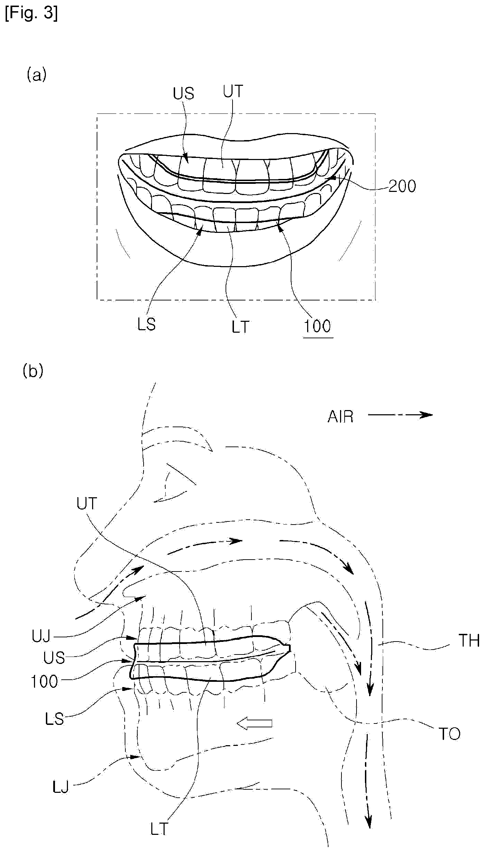

Fig. 1 includes perspective views of an oral appliance according to a first exemplary embodiment, in which (a) is a top perspective view and (b) is a bottom perspective view, Fig. 2 is a cross-sectional view taken along line A-A' in (a) of Fig. 1, and Fig. 3 is a view illustrating the oral appliance worn in an oral cavity according to the first exemplary embodiment, in which (a) is a front view and (b) is a side view.

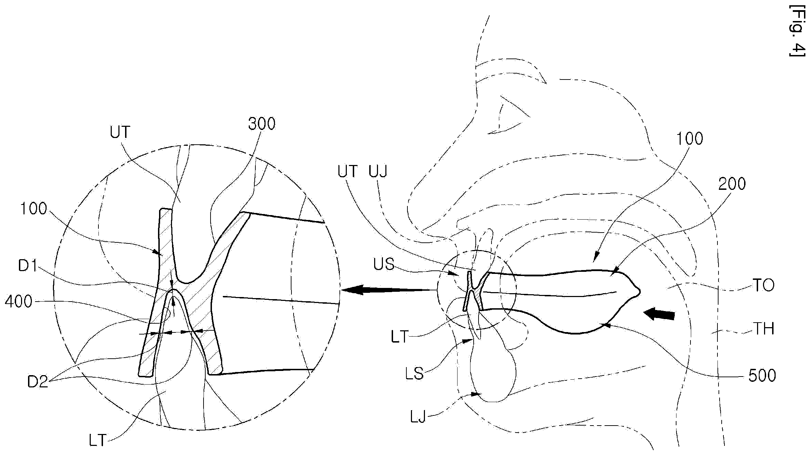

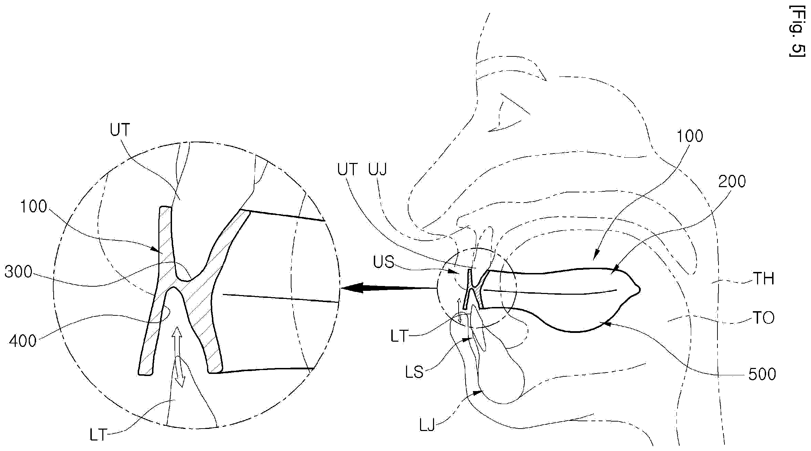

Fig. 4 is a cross-sectional view and a partially enlarged cross-sectional view of (b) of Fig. 3, and Fig. 5 is a cross-sectional view and a partially enlarged cross-sectional view illustrating a usage state of the oral appliance according to the first exemplary embodiment.

An oral appliance may include a body 200, an upper teeth recess 300, a lower teeth recess 400, and a guide part 500 as shown in Figs. 1 and 2.

As illustrated in Fig. 1, the body 200 may have a shape corresponding to teeth arrangements US and LS. Namely, the body 200 may have a horseshoe shape. Thus, the teeth arrangement US of an upper jaw UJ and the teeth arrangement LS of a lower jaw LJ may correspond to the body 200.

As illustrated in Figs. 3 and 4, teeth UT included in the teeth arrangement US of the upper jaw UJ may be inserted into the upper teeth recess 300 formed in an upper portion of the body 200 as described hereinafter, and teeth LT included in the teeth arrangement LS of the lower jaw LJ may be inserted into the lower teeth recess 400 formed in a lower portion of the body 200 as described hereinafter.

A material of the body 200 is not particularly limited and may be any material as long as it does not pose a threat when the body 200 formed of the material is applied to an oral cavity.

In order to manufacture the oral appliance 100 including the body 200, any known method may be used, including but not limited to: forming a model of the teeth UT and LT of a user and subsequently shaping the oral appliance 100 appropriately based on the model, taking an image of the teeth UT and LT of the user and manufacturing the oral appliance 100 by using a 3D printer, or the like.

The upper teeth recess 300 may be formed in an upper portion of the body 200. As illustrated in Figs. 3 through 5, the teeth UT included in the teeth arrangement US of the upper jaw UJ may be inserted into the upper teeth recess 300. To this end, the upper teeth recess 300 may have a shape corresponding to the teeth arrangement US of the upper jaw UJ on the whole, namely, a horseshoe shape as mentioned above; also, the upper teeth recess 300 may have a shape corresponding to each tooth UT included in the teeth arrangement US of the upper jaw UJ.

A method for forming the upper teeth recess 300 in the upper portion of the body 200 is not particularly limited, and any method, such as forming a model of the teeth UT of a user and subsequently shaping the upper teeth recess 300 to correspond thereto or forming the upper teeth recess 300 by using a 3D printer, or the like, may be used.

The teeth UT of the upper jaw UJ may be fixed to the upper teeth recess 300 through friction. Namely, as mentioned above, the upper teeth recess 300 may be formed to have a shape corresponding to the teeth UT of the upper jaw UJ such that there is no gap between the upper teeth recess 300 and the teeth UT of the upper jaw UJ.

Thus, the teeth UT of the upper jaw UJ may be tightly inserted into the upper teeth recess 300 and fixed to the upper teeth recess 300 through friction. Unless an external force stronger than frictional force between the upper teeth recess 300 and the teeth UT of the upper jaw UJ is applied, the teeth UT of the upper jaw UJ will not be separated from the upper teeth recess 300.

The lower teeth recess 400 may be formed in a lower portion of the body 200. As illustrated in Figs. 3 through 5, the teeth LT included in the teeth arrangement LS of the lower jaw LJ may be inserted into the lower teeth recess 400. To this end, the lower teeth recess 400 may have a shape corresponding to the teeth arrangement LS of the lower jaw LJ on the whole, namely, a horseshoe shape as mentioned above; also, the lower teeth recess 400 may have a shape corresponding to each tooth LT included in the teeth arrangement LS of the lower jaw LJ.

The method for forming the lower teeth recess 400 in the lower portion of the body 200 is not particularly limited, and any method may be used, including, but not limited to, forming a model of the teeth LT of a user and subsequently shaping the lower teeth recess 400 to generally correspond thereto, taking an image of the teeth LT of a user and forming the lower teeth recess 400 based on the image by using a 3D printer, or the like.

When the teeth LT of the lower jaw LJ is inserted into the lower teeth recess 400, the lower jaw LJ may move forward by a predetermined distance.

Namely, as illustrated in Figs. 3 and 4, the lower jaw LJ may move forward by a predetermined distance A such that front teeth of the lower jaw LJ are positioned in front of the front teeth of the upper jaw UJ by a predetermined distance. Accordingly, a tongue TO, being attached to the lower jaw LJ, may also move forward along with the lower jaw LJ, as illustrated.

To this end, as mentioned above, the lower teeth recess 400 may be formed in the body 400 such that when the teeth LT of the lower jaw LJ is inserted, the teeth LT of the lower jaw LJ move forward relative to a natural position, such that the front teeth of the lower jaw LJ are positioned in front of the front teeth of the upper jaw UJ by a predetermined distance.

Meanwhile, as illustrated in Fig. 4, when the teeth LT of the lower jaw LJ are inserted into the lower teeth recess 400, the teeth LT of the lower jaw LJ may be spaced apart from the lower teeth recess 400 at predetermined gaps D 1 and D2 therebetween in vertical and horizontal directions.

With the presence of the gaps D1 and D2 between the lower teeth recess 400 and the teeth LT of the lower jaw LJ in the vertical and horizontal directions, as illustrated in Fig. 5, the teeth LT of the lower jaw LJ may be released from the lower teeth recess 400 and moved downwards as needed; accordingly, the lower jaw LJ may also be moved downwards.

Thus, since the lower jaw LJ may naturally move downwards, stiffness of the neighboring muscles and ligaments occurred by, for example, the forward movement of the lower jaw LJ and maintenance of this state during sleep, may be resolved and relaxed.

The foregoing downward movement of the lower jaw LJ may be stopped when the guide part 500 comes into contact with a lingual mucous membrane of the lower jaw LJ. When this state is maintained, the neighboring muscles and ligaments may stiffen again.

In order to resolve this, the lower jaw LJ may naturally move upwards, namely, may return to a position before the movement, in which the teeth LT of the lower jaw LJ are inserted into the lower teeth recess 400 and the lower jaw LJ moves forward.

The downward movement of the lower jaw LJ from the oral appliance 100 and the upward movement thereof to the oral appliance 100 may be repeated while the user, wearing the oral appliance 100, is sleeping.

Thus, even if the user wears the oral appliance 100 for a long period of time , the muscles and ligaments around the oral cavity may not be stiffened. Also, pain, malocclusion, or a TMJ disorder caused by rigidity of the muscles and ligaments around the oral cavity may not occur. Also, since air flow through the airway is improved, rhinitis or rhinosinusitis may be mitigated.

The gap D1 between the teeth LT of the lower jaw LJ and the lower teeth recess 400 in the vertical direction may range from 0.5 mm to 2 mm.

According to released research, a natural gap between the teeth UT of the upper jaw UJ and the teeth LJ of the lower jaw LJ, for example, the gap during sleep, ranges approximately from 2 mm to 4 mm.

Meanwhile, a distance between the upper teeth recess 300 and the lower teeth recess 400 is approximately 1.5 mm. Thus, if the gap D1 between the lower teeth recess 400 and the teeth LT of the lower jaw LJ in the vertical direction is smaller than 0.5 mm, a distance between the teeth UT of the upper jaw UJ and the teeth LT of the lower jaw may be smaller than 2 mm to 4 mm, the natural gap.

In this case, the brain may misunderstand the body 200 interposed between the teeth UT of the upper jaw UJ and the teeth LT of the lower jaw LJ to be food, causing masticatory movements to be made by the related muscles and ligaments. Force exerted by the masticatory movements may be transmitted to the teeth UT and LT of the upper and lower jaws UJ and LJ through the oral appliance 100.

The force transmitted to the teeth UT and LT may break the teeth UT and LT or periodontal ligaments and destroy an alveolar bone. Also, the teeth UT and LT may be lost or periodontitis may develop. Namely, the teeth UT and LT may be damaged.

In addition, the force exerted by the masticatory movements may spread to the muscles, ligaments, and TMJ around the oral cavity having the oral appliance 100 installed therein, damaging the muscles and ligaments. As a result, malocclusion or a TMJ disorder, as along with pain, may occur.

If the gap D1 between the lower teeth recess 400 and the teeth LT of the lower jaw LJ in the vertical direction exceeds 2 mm, a distance between the teeth UT of the upper jaw UJ and the teeth LT of the lower jaw may be greater than 4 mm, the natural gap.

Thus, the related muscles and ligaments may be burdened and stiffened. Also, pain, malocclusion, and a TMJ disorder may occur.

Therefore, the gap D1 between the lower teeth recess 400 and the teeth LT of the lower jaw LJ in the vertical direction ranging from 0.5 mm to 2 mm is appropriate.

Meanwhile, the gap D2 between the lower teeth recess 400 and the teeth LT of the lower jaw LJ in the horizontal direction may range from 0.3 mm to 0.5 mm. As discussed above, since the gap D2 between the lower teeth recess 400 and the teeth LT of the lower jaw LJ in the horizontal direction on one side of the lower teeth recess 400 ranges from 0.3 mm to 0.5 mm, a total gap in both sides may range from 0.6 mm to 1.0 mm.

If the gap D2 between the lower teeth recess 400 and the teeth LT of the lower jaw LJ in the horizontal direction on one side of the lower teeth recess 400 is smaller than 0.3 mm, the teeth LT of the lower jaw LJ may occasionally be fixed to the lower teeth recess 400 through friction. Also, the teeth LT of the lower jaw LJ may be hindered from being released and moving downwards from the lower teeth recess 400, when needed.

Thus, the related muscles and ligaments stiffened due to the forward movement of the lower jaw LJ may not be relaxed, causing pain, discomfort, malocclusion, or a TMJ disorder.

If the gap D2 between the lower teeth recess 400 and the teeth LT of the lower jaw LJ in the horizontal direction on one side of the lower teeth recess 400 exceeds 0.5 mm, the teeth LT of the lower jaw LJ may be freely moved within the lower teeth recess 400, but the teeth LT of the lower jaw LJ may be completely released and separated from the lower teeth recess 400.

Thus, the lower jaw LJ may not be maintained in a state in which it is placed forward by a predetermined distance, losing the ability to prevent snoring or sleep apnea.

Therefore, the gap D2 between the lower teeth recess 400 and the teeth LT of the lower jaw LJ in the horizontal direction on one side of the lower teeth recess 400 ranging from 0.3 mm to 0.5 mm is appropriate.

The range of the gaps D1 and D2 between the lower teeth recess 400 and the teeth LT of the lower jaw LJ in the vertical or horizontal direction may be modified to be individually applied by adding or subtracting a value from a reference value of a portion or entirety of the lower teeth recess 400 according to a tilt angle of the occlusal plane, a degree of protrusion of teeth, an inclination state of teeth in forward/backward and inward/outward directions, or a degree of teeth deviating from the overall teeth arrangement, of each of the users.

Also, as illustrated in Fig. 4, the lower teeth recess 400 may be spaced apart from the teeth LT of the lower jaw LJ in the horizontal direction forwardly and backwardly. In addition, the lower teeth recess 400 may also be spaced apart from the teeth LT of the lower jaw LJ in the horizontal direction left and right.

As illustrated in Figs. 1 and 2, the guide part 500 may extend downwards from the body 200. For example, the guide part 500 may extend downwards by 3 mm to 5 mm from the body 200. However, the length of the guide part 500 extending from the body 200 may not be particularly limited and any length may be applied according to a state of an oral cavity of individuals.

As described above, as the teeth LT of the lower jaw LJ move downwards from the lower teeth recess 400 to relax the related muscles and ligaments stiffened as the lower jaw LJ moves forward and maintained in the state of being ahead by a predetermined distance, the lower jaw LJ may also be moved downwards by a predetermined distance naturally.

Such movement of the lower jaw LJ may be stopped when the guide part 500 comes into contact with the lingual mucous membrane of the lower jaw LJ, and the lower jaw LJ may be maintained in that state.

When the lower jaw LJ is moved or maintained in a moved state to relax the stiffened muscles and ligaments, other normal muscles or ligaments may be stiffened. In order to relax the other stiffened muscles and ligaments, the lower jaw LJ may be naturally moved upwards as illustrated in Fig. 5, and the teeth LT of the lower jaw LJ may be inserted into the lower teeth recess 400.

At this time, the guide part 500 may guide the teeth LT of the lower jaw LJ to be easily and properly inserted into the lower teeth recess 400.

To this end, the guide part 500 may extend from an inner side of the body 200 as illustrated in Figs. 1 and 2. Also, the guide part 500 may extend to cover a portion of a gum part of the molars among the teeth LT of the lower jaw LJ.

However, the shape of the guide part 500 is not particularly limited and the guide part 500 may have any shape as long as it has the aforementioned functions.

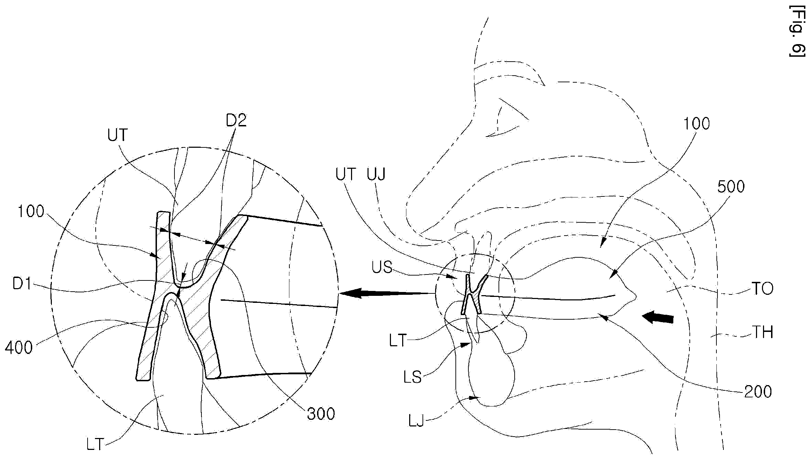

Fig. 6 is a cross-sectional view and a partially enlarged cross-sectional view illustrating a wearing state of the oral appliance according to a second exemplary embodiment. The oral appliance 100 according to the second exemplary embodiment is different from that of the first exemplary embodiment described above with reference to Figs. 1 through 5 in that, when the oral appliance is worn, the teeth LT of the lower jaw LJ are fixed in the lower teeth recess 400 through friction, the upper teeth recess 300 is spaced apart from the teeth UT of the upper jaw UJ at predetermined intervals D3 and D4 in vertical and horizontal directions, and guide parts 500 extends upwards from the body 200. Thus, different parts and components will mainly be described, and the other components may simply refer back to the above descriptions with reference to Figs. 1 through 5.

As illustrated in Fig. 6, in the oral appliance 100 according to the second exemplary embodiment, the teeth UT of the lower jaw LJ may be fixed in the lower teeth recess 400. Namely, the lower teeth recess 400 may be formed to correspond to the teeth LT of the lower jaw LJ such that there is no gap between the lower teeth recess 400 and the teeth LT of the lower jaw LJ.

Thus, the teeth LT of the lower jaw LJ may be tightly inserted into the lower teeth recess 400 and fixed through friction. Also, the teeth LT of the lower jaw LJ will not be separated from the lower teeth recess 400 unless an external force greater than frictional force between the lower teeth recess 400 and the teeth LT of the lower jaw LJ is applied.

The upper teeth recess 300 may be spaced apart from the teeth UT of the upper jaw UJ at predetermined intervals D1 and D2 in the vertical and horizontal directions.

The guide part 500 may extend upwards from the body 200.

Through this configuration, as the teeth UT of the upper jaw UJ are relatively moved in the upper teeth recess 300 in order to relax related muscles and ligaments stiffened due to a forward movement of the lower jaw LJ, the lower jaw LJ may naturally move downwards together with the oral appliance 100.

While the lower jaw LJ is moving downwards together with the oral appliance 100, if the guide part 500 comes into contact with the molars among the teeth UT of the upper jaw UJ released from the upper teeth recess 300, the lower jaw LJ may stop moving and be maintained in the same state.

In this state, in order to relax other stiffened muscles and ligaments, the lower jaw LJ may naturally move upwards together with the oral appliance 100 to the position, namely, to the position prior to being released, in which the teeth UT of the upper jaw UJ are inserted into the upper teeth recess 300 and the lower jaw LJ moves forward.

At this time, the guide part 500 may guide the teeth UT of the upper jaw UJ to be easily and properly inserted into the upper teeth recess 300.

The reciprocating movement of the lower jaw LJ may be repeated while the user wearing the oral appliance 100 is sleeping.

Thus, as described above, the related muscles and ligaments stiffened due to the forward movement of the lower jaw and the movement of the lower jaw may be relaxed, and thus, even though the user wears the oral appliance, pain, malocclusion, or a TMJ disorder may not occur. Also, since air flow through the airway is facilitated, rhinitis or rhinosinusitis may be mitigated.

Meanwhile, the gap D1 between the upper teeth recess 300 and the teeth UT of the upper jaw UJ in the vertical direction may range from 0.5 mm to 2 mm. Also, the gap D2 between the upper teeth recess 300 and the teeth UT of the upper jaw UJ in the horizontal direction in one side thereof may range from 0.3 mm to 0.5 mm.

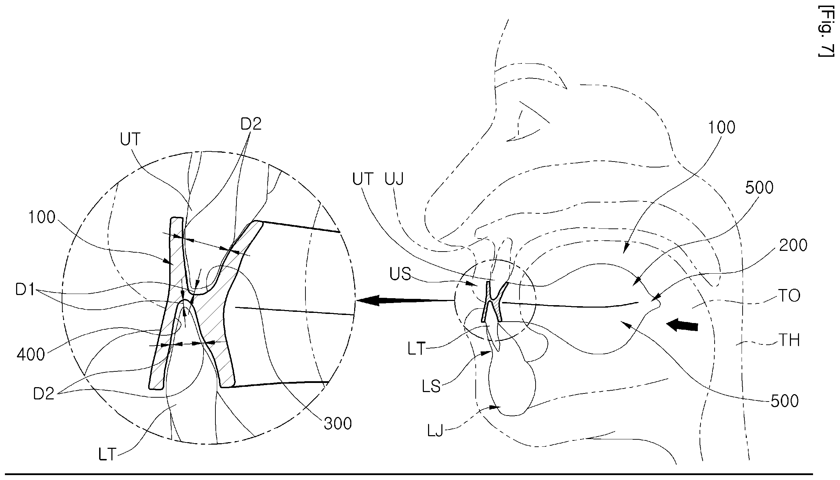

Fig. 7 is a cross-sectional view and a partially enlarged cross-sectional view illustrating a wearing state of an oral appliance according to a third exemplary embodiment.

The oral appliance 100 according to the third exemplary embodiment is different from that of the first exemplary embodiment, described above with reference to Figs. 1 through 5, in that the upper teeth recess 300 is spaced apart from the teeth UT of the upper jaw UJ at predetermined intervals D1 and D2 in vertical and horizontal directions, the lower teeth recess 400 is spaced apart from the teeth UT of the lower jaw LJ at predetermined intervals D1 and D2 in vertical and horizontal directions, and the guide part 500 extends downwards and upwards from the body 200.

Thus, different parts and components will mainly be described, and the other components may simply refer back to the above descriptions with reference to Figs. 1 through 5.

As illustrated in Fig. 7, in the oral appliance 100 according to the third exemplary embodiment, the upper teeth recess 300 may be spaced apart from the teeth UT of the upper jaw UJ at predetermined intervals D1 and D2 in the vertical and horizontal directions, and lower teeth recess 400 may be spaced apart from the teeth LT of the lower jaw LJ at predetermined intervals D1 and D2 in the vertical and horizontal directions.

Also, the guide part 500 may extend both upwards and downwards by a predetermined distance, from the body 200.

The oral appliance 100 according to the third exemplary embodiment may be used in a case in which a state of the teeth arrangements US and LS of the upper and lower jaws UJ and LJ or a state of the teeth UT and LT included therein is so poor, such that the teeth may be lost if fixed to the upper teeth recess 300 or the lower teeth recess 400 through friction, in a case in which a length of the crowns of the upper and lower jaws is too short to obtain sufficient frictional force.

In this configuration, when any one of the teeth UT of the upper jaw UJ and the teeth LT of the lower jaw LJ is more tightly attached to the upper teeth recess 300 or the lower teeth recess 400 according to posture during sleep, a side less tightly attached may be moved within the upper teeth recess 300 or the lower teeth recess 400. Thus, the lower jaw LJ may be naturally moved to relax the stiffened muscles and ligaments.

For example, if the teeth UT of the upper jaw UJ are more tightly attached to the upper teeth recess 300, the lower jaw LJ may perform a reciprocating movement in a manner identical to that of the oral appliance 100 according to the first exemplary embodiment as described above, and if the teeth UT of the lower jaw LJ are more tightly attached to the lower teeth recess 400, the lower jaw LJ may perform a reciprocating movement in a manner identical to that of the oral appliance 100 according to the second exemplary embodiment as described above.

The reciprocating movement of the lower jaw LJ may be repeated while the user wearing the oral appliance 100 is sleeping.

Thus, as described above, the related muscles and ligaments stiffened due to the forward movement of the lower jaw and the other movement of the lower jaw may be relaxed, and thus, even though the user wears the oral appliance, pain, malocclusion, or a TMJ disorder may not occur. Also, since air flow through the airway is facilitated, rhinitis or rhinosinusitis may be mitigated.

In this case, the gap D1 between the upper teeth recess 300 and the teeth UT of the upper jaw UJ in the vertical direction and the gap D1 between the lower teeth recess 400 and the teeth LT of the lower jaw LJ in the vertical direction may range from 0.5 mm to 2 mm.

Also, the gap D2 between the upper teeth recess 300 and the teeth UT of the upper jaw UJ in the horizontal direction, and the gap D2 between the lower teeth recess 400 and the teeth LT of the lower jaw LJ in the horizontal direction may range from 0.3 mm to 0.5 mm.

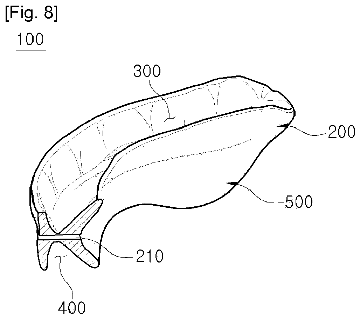

Fig. 8 is a perspective cross-sectional view illustrating an oral appliance according to a fourth exemplary embodiment.

The oral appliance 100 according to the fourth exemplary embodiment is different from the oral appliance 100 described above with reference to Figs. 1 through 5, in that a vent hole 210 is formed in the body 200 of the oral appliance 100.

Thus, different parts and components will mainly be described, and the other components may simply refer back to the above descriptions with reference to Figs. 1 through 5.

As illustrated in Fig. 8, in the oral appliance 100 according to the fourth exemplary embodiment, the vent hole 210 may be formed in the body 200. The vent hole 210 may be formed in a central portion of the body 200 between the upper teeth recess 300 and the lower teeth recess 400.

Thus, the user may breathe through his or her mouth through the vent hole 210 of the body 200 while wearing the oral appliance 100.

Fig. 9a is a view illustrating an embodiment regarding an oral appliance fixed to lower jaw teeth.

With reference to Figs. 1 to 8, an oral appliance which is fixed to the upper jaw teeth (molar) has been described above. As illustrated in Fig. 9a, the oral appliance fixed to lower jaw teeth (molar) may also be possible.

As illustrated in Fig. 9a, an oral appliance 60 fixed to lower jaw teeth is similar to an oral appliance 100 fixed to upper jaw teeth when viewed from front of the teeth. However, as described below, the oral appliance fixed to lower jaw teeth 60 has various characteristics different from those of the oral appliance fixed to upper jaw teeth 100.

Fig. 9b is a side view illustrating the oral appliance fixed to lower jaw teeth according to the embodiment illustrated in Fig. 9a.

As illustrated in Fig. 9b, one most noticeable feature of the oral appliance fixed to lower jaw teeth 60 is that the oral appliance 60 is fixed to a portion of the lower jaw teeth LT of a user. Particularly, at least one lower jaw tooth LT in the left and right molar regions among the lower jaw teeth LT of a user is fixed to a lower molar fixing recess 810 by a frictional force.

Meanwhile, the upper jaw teeth arrangement US is not fixed to the oral appliance fixed to lower jaw teeth 60. Accordingly, the upper jaw teeth arrangement US may freely move within a predetermined range. Further, an upper teeth guide part 710 is formed so that even if the upper jaw teeth arrangement US moves, the upper jaw teeth arrangement US may be positioned in place inside an upper teeth recess 700 of a body 600. The upper teeth guide part 710 is formed to contact a side surface of the upper law teeth.

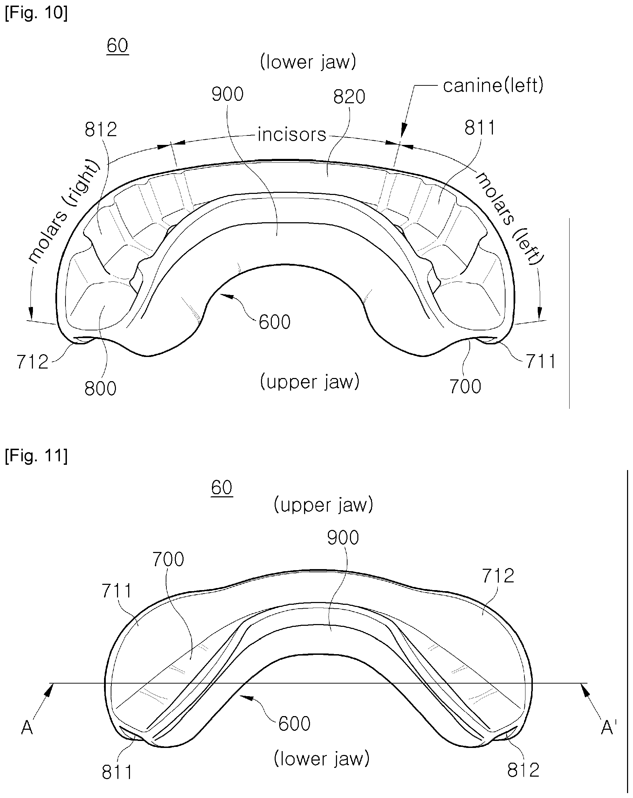

Fig. 10 is a rear view illustrating a bottom surface of the oral appliance fixed to lower jaw teeth according to the embodiment illustrated in Fig. 9a, and Fig. 11 is a rear view illustrating an upper surface of the oral appliance fixed to lower jaw teeth according to the embodiment illustrated in Fig. 9a.

Each part constituting the oral appliance 60 fixed to lower jaw teeth will be described in more detail with reference to Figs. 10 and 11.

The oral appliance 60 fixed to lower jaw teeth is configured by forming a body 600 with a material, such as a medical resin and other material that is safe for human body. The body 600 further comprises an upper teeth recess 700, an upper teeth guide part 710, a lower teeth recess 800, and a tongue tip mounting part 900.

Meanwhile, the lower teeth recess 800 further comprises lower molar fixing recesses 811 and 812 in which a molar portion of lower teeth is positioned and a lower incisor recess 820 in which an incisor portion of lower teeth is positioned.

At least one of the left and right molars of lower jaw teeth is strongly engaged with the lower molar fixing recesses 811 and 812 by a frictional force. On the other hand, the incisor portion of the lower jaw teeth is positioned in the lower incisor recess 820, but the incisor portion is not engaged with an inner side of the lower incisor recess 820.

A boundary between the lower molar fixing recess 811 and the lower incisor recess 820 is a left canine, and a boundary between the lower molar fixing recess 812 and the lower incisor recess 820 is a right canine.

Meanwhile, the upper jaw teeth UT and the lower jaw teeth LT respectively are positioned in the upper teeth recess 700 and the lower teeth recess 800. Here, the lower teeth recess 800 is formed to be positioned ahead of the normal occlusal surface of a user so that the lower jaw teeth LT of the user wearing the oral appliance 60 move forward further than before wearing the oral appliance. As the user's lower jaw teeth LT move forward, the cross-sectional area of the user's airway widens, improving the air flow during respiration. This helps prevent or alleviate snoring while the user sleeps. It also helps to relieve the user of inflammation, such as rhinitis and sinusitis.

Upper teeth guide parts 711 and 712 are formed so that the upper jaw teeth arrangement US may be located in place in the upper teeth recess 700 of the body 600 even if the upper jaw teeth arrangement US moves.

As described above, the upper jaw teeth arrangement US is strongly engaged with the upper teeth recess 700 or is not fixed to the inside of the upper teeth recess 700. Accordingly, the upper jaw teeth arrangement US may freely move within a predetermined range departing from the upper teeth recess 700. Thus, although the oral appliance 60 is worn for a long time, muscles and ligaments around the oral cavity may not be rigid. Further, pain, malocclusion, or temporomandibular joint disorders caused by the stiffness of muscles and ligaments around oral cavity may not occur.

However, since the upper jaw teeth arrangement US should be able to locate in the upper teeth recess 700, the upper teeth guide parts 711 and 712 are formed to extend from the body 600 at a portion where the upper teeth guide parts 711 and 712 contact a side surface of an upper jaw teeth UT. Further, the upper teeth guide parts 711 and 712 may be formed such that an extended length of an upper molar region is larger than an extended length of an upper incisor region.

Further, the tongue tip mounting part 900 is formed as a cavity so that a tongue tip of a user may stably be seated ahead of the oral appliance 60.

Various conventional oral devices, such as an oral appliance for preventing snoring, do not provide a space for accommodating the user's tongue. The user is unable to stably rest the tongue, and thus the tongue moves back to the original rear position, and the adaptability to the oral appliance is lowered.

In order to solve such a problem, an oral appliance fixed to lower jaw teeth 60 comprises the tongue tip mounting part 900 allowing the user's tongue tip to be stably placed ahead.

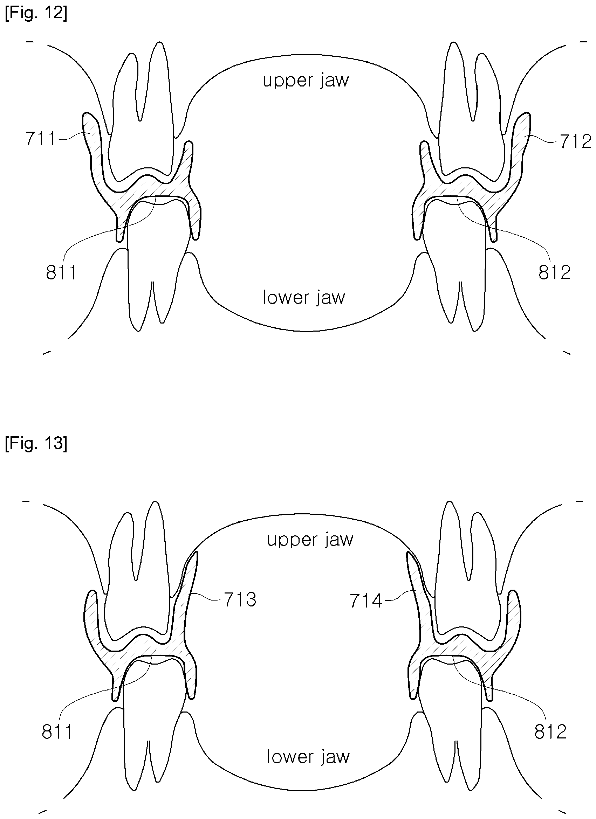

Figs. 12 to 14 show three types of cross-sectional views illustrating a molar portion of the oral appliance fixed to lower jaw teeth according to the embodiment illustrated in Fig. 9a,

Each of Figs. 12 to 14 is a cross-sectional view illustrating a plane in the normal direction of a front side of a user, which is one of planes including the straight line connecting a left-side molar portion (A' point) and right-side molar portion (A point) of the user in Fig. 11.

As illustrated in Fig. 12, the lower jaw left-side molar and the lower jaw right-side molar, respectively, are positioned in inner sides of lower molar fixing recesses 811 and 812. Further, at least one lower jaw molar is strongly engaged with the inner side of the lower molar fixing recesses 811 and 812 to prevent the oral appliance 60 from too easily escaping off from a user.

As described above, upper jaw molars are positioned in an upper teeth recess 700, but are not strongly engaged with an inner side of the upper teeth recess 700. Further, the upper teeth guide parts 711 and 712, respectively, are formed in the right and left of the upper teeth recess 700 of the body 600 so that upper jaw molars may escape off from the upper teeth recess 700 and then come back to their position in the upper teeth recess 700.

Further, as illustrated in Fig. 13, upper teeth guide parts 713 and 714, respectively, are formed in the right and left of the upper teeth recess 700 of the body 600 so that upper jaw molars may escape off from the upper teeth recess 700 and then come back to their sites in the upper teeth recess 700.

In other words, the upper teeth guide parts 713 and 714 are formed to extend from the body 600 toward the palate of the user to face an inner side of upper jaw teeth.

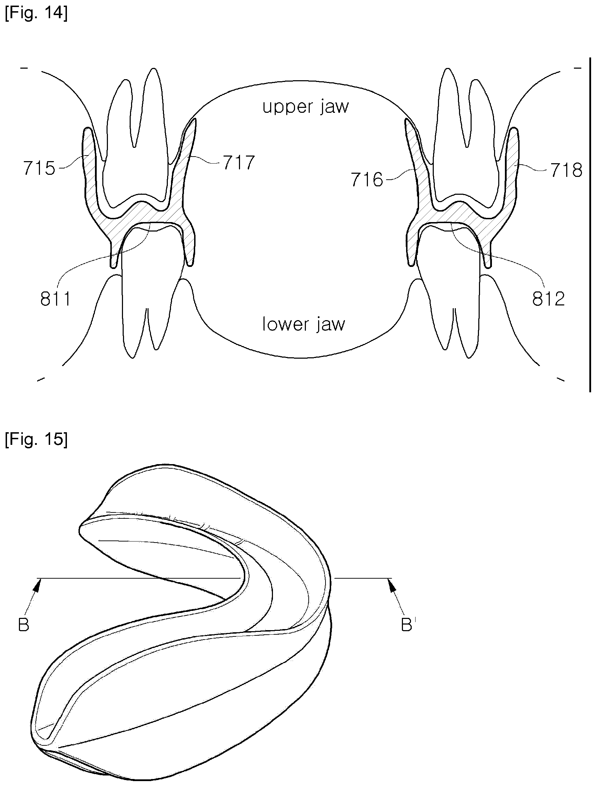

Further, as illustrated in Fig. 14, upper teeth guide parts 715, 716, 717 and 718, respectively, may be formed in the right and left of the upper teeth recess 700 of the body 600 so that upper jaw molars may escape off from the upper teeth recess 700 and then come back to their position in the upper teeth recess 700.

In other words, first upper teeth guide parts 715 and 718 disposed to face an outer side surface of upper jaw teeth are formed to extend from a body 600 to a space between a cheek and an upper jaw teeth of a user. Further, second upper teeth guide parts 716 and 717 disposed to face an inner side of upper jaw teeth are formed to extend from the body 600 toward a palate of a user.

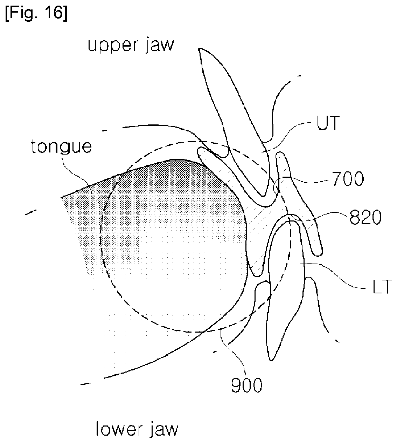

Fig. 15 is a view from the front right, illustrating an upper surface of the oral appliance fixed to lower jaw teeth according to the embodiment illustrated in Fig. 9a, and Fig. 16 is a view illustrating the relationship among a tongue, teeth, the oral appliance fixed to lower jaw teeth, and a cross-section of an anterior teeth part of the oral appliance fixed to lower jaw teeth in a cross-section illustrated in Fig. 15.

The cross-sectional view of Fig. 16 is a cross-sectional view illustrating a plane including the straight line connecting front and rear portions of a user among planes including the straight line connecting an upper incisor portion (B point) and a lower incisor portion (B' point) shown in Fig. 15.

As illustrated in Fig. 16, the upper jaw incisor UT is positioned in the upper teeth recess 700, and the lower jaw incisor LT is positioned in the lower incisor recess 820.

Neither the upper teeth recess 700 nor the lower incisor recess 820 engages strongly with teeth. Further, as described above referring to Figs. 10 and 11, the upper jaw incisor UT and the lower jaw incisor LT respectively are positioned in the upper teeth recess 700 and the lower incisor recess 820. Here, the lower teeth recess 800 is formed to be positioned ahead of the normal occlusal surface of a user so that the lower jaw teeth LT of the user wearing the oral appliance 60 move forward further than before wearing the oral appliance. As the user's lower jaw teeth LT moves forward, the cross-sectional area of the user's airway is widened, so that the air flow during respiration is improved.

Further, a user's tongue is placed on the tongue tip mounting part 900 formed on the body 600. It is preferable that the surface of the tongue tip mounting part 900 is smoothed so as not to cause inconvenience or discomfort to a user. Furthermore, it is preferable that the tongue tip mounting part 900 is formed to be curved along with curve of the tongue tip.

As described above, according to the present invention, although when wearing an oral appliance fixed to lower jaw teeth, it is possible to implement an oral appliance that allows lower jaw teeth naturally to move.

Further, it is possible to implement an oral appliance that allows the related muscles and ligaments stiffness caused by forward advancement of the low jaw to be relaxed by permitting movement of the low jaw.

Furthermore, it is possible to implement an oral appliance that enables a pain, a malocclusion or a jaw joint disorder not to occur even when an oral appliance is worn.

Accordingly, since air flow through an airway of a user is more facilitated, symptoms of snoring, sleep apnea, rhinitis, or rhinosinusitis may be improved.

## Claims

1. An oral appliance fixable to lower jaw teeth of a patient comprising:
a body (200) having a shape corresponding to a teeth arrangement;
a lower teeth recess (400) formed in a lower portion of the body to allow the lower jaw teeth to be inserted therein;
an upper teeth recess (300) formed in an upper portion of the body to allow upper jaw teeth to be inserted therein and allowing the lower jaw to move backward by a predetermined distance; and
an upper teeth guide part (710) formed to extend from the body to face a side surface of the upper jaw teeth,
wherein the lower teeth recess further comprises a lower molar fixing recess (810) formed to strongly engage with at least a portion of left and right molars of the lower jaw teeth by a frictional force and a lower incisor recess (820) formed to allow other lower jaw teeth except the left and right molars to position therein,
wherein the lower incisor recess (820) is formed to be spaced apart from lower incisors, and thus the lower incisor recess does not contact to the lower incisors,
wherein the upper teeth recess (300) is formed to be spaced apart from upper jaw teeth, and thus the upper teeth recess does not strongly engage with the upper jaw teeth,
wherein the upper teeth recess (300) and the lower teeth recess (400) are formed such that the lower jaw teeth of the patient move forward further than before wearing the oral appliance fixed to the lower jaw teeth.

2. The oral appliance of claim 1, further comprising a tongue tip mounting part (900) formed on a portion of the body to allow the patient's tongue to be placed thereon.

3. The oral appliance of claim 1, wherein a boundary between the lower molar fixing recess (810) and the lower incisor recess (820) is a canine.

4. The oral appliance of claim 1, wherein the upper teeth guide part (710) is formed such that an extended length of an upper molar region is larger than an extended length of an upper incisor region.

5. The oral appliance of claim 4, wherein the upper teeth guide part (710) is formed to extend from the body (200) to a space between a cheek and an upper jaw teeth of the patient to face an outer side of the upper jaw teeth.

6. The oral appliance of claim 4, wherein the upper teeth guide part (710) is formed to extend toward a palate of the patient to face an inner side of the upper jaw teeth.

7. The oral appliance of claim 4, wherein the upper teeth guide part (710) comprises:
a first upper teeth guide part formed to extend from the body to a space between a cheek and an upper jaw teeth of the patient to face an outer side of the upper jaw teeth; and
a second upper teeth guide part formed to extend from the body toward the palate of the patient to face an inner side of the upper jaw teeth.

8. The oral appliance of claim 1, wherein the body (200) is made of a medical resin or other human-safe material.

9. The oral appliance of claim 1, wherein the lower teeth recess (300) is formed such that the depth of the lower teeth recess is not larger than a height of an exposed surface of the lower jaw teeth of the patient.

## Patentansprüche

1. Orale Vorrichtung, die an Unterkieferzähnen eines Patienten fixierbar ist, umfassend:
einen Körper (200), der eine Form entsprechend einer Zahnanordnung aufweist;
eine untere Zahnaussparung (400), die in einem unteren Abschnitt des Körpers gebildet ist, um den Unterkieferzähnen zu erlauben, darin eingesetzt zu werden;
eine obere Zahnaussparung (300), die in einem oberen Abschnitt des Körpers gebildet ist, um Oberkieferzähnen zu erlauben, darin eingesetzt zu werden, und dem Unterkiefer zu erlauben, sich um eine vorgegebene Distanz nach hinten zu bewegen; und
ein oberes Zahnführungsteil (710), das gebildet ist, sich so von dem Körper zu erstrecken, dass es zu einer Seitenoberfläche der Oberkieferzähne zeigt,
wobei die untere Zahnaussparung weiter eine untere Molarfixieraussparung (810) umfasst, die ausgebildet ist, fest mit mindestens einem Abschnitt linker und rechter Molare der Unterkieferzähne durch eine Reibungskraft einzugreifen, und eine untere Schneidezahnaussparung (820), die gebildet ist, anderen Unterkieferzähnen außer den linken und rechten Molaren zu erlauben, darin positioniert zu werden,
wobei die untere Schneidezahnaussparung (820) gebildet ist, von unteren Schneidezähnen beabstandet zu sein, und daher die untere Schneidezahnaussparung die unteren Schneidezähne nicht berührt,
wobei die obere Zahnaussparung (300) gebildet ist, von Oberkieferzähnen beabstandet zu sein, und daher die obere Zahnaussparung nicht fest mit den Oberkieferzähnen eingreift,
wobei die obere Zahnaussparung (300) und die untere Zahnaussparung (400) so gebildet sind, dass die Unterkieferzähne des Patienten sich weiter nach vorne bewegen als vor dem Tragen der oralen Vorrichtung, die an den Unterkieferzähnen fixiert ist.

2. Orale Vorrichtung nach Anspruch 1, weiter umfassend ein Zungenspitzenhalteteil (900), das an einem Abschnitt des Körpers gebildet ist, um der Zunge des Patienten zu erlauben, darauf platziert zu werden.

3. Orale Vorrichtung nach Anspruch 1, wobei eine Grenze zwischen der unteren Molarfixieraussparung (810) und der unteren Schneidezahnaussparung (820) ein Eckzahn ist.

4. Orale Vorrichtung nach Anspruch 1, wobei das obere Zahnführungsteil (70) so gebildet ist, dass eine Erstreckungslänge eines oberen Molarbereichs größer als eine Erstreckungslänge eines oberen Schneidezahnbereichs ist.

5. Orale Vorrichtung nach Anspruch 4, wobei das obere Zahnführungsteil (710) gebildet ist, sich von dem Körper (200) zu einem Raum zwischen einer Backe und Oberkieferzähnen des Patienten zu erstrecken, um zu einer Außenseite der Oberkieferzähne zu zeigen.

6. Orale Vorrichtung nach Anspruch 4, wobei das obere Zahnführungsteil (710) gebildet ist, sich zu einem Gaumen des Patienten zu erstrecken, um zu einer Innenseite der Oberkieferzähne zu zeigen.

7. Orale Vorrichtung nach Anspruch 4, wobei das obere Zahnführungsteil (710) umfasst:
ein erstes oberes Zahnführungsteil, das gebildet ist, sich von dem Körper zu einem Raum zwischen einer Backe und Oberkieferzähnen des Patienten zu erstrecken, um zu einer Außenseite der Oberkieferzähne zu zeigen; und
ein zweites oberes Zahnführungsteil, das gebildet ist, sich von dem Körper zu dem Gaumen des Patienten zu erstrecken, um zu einer Innenseite der Oberkieferzähne zu zeigen.

8. Orale Vorrichtung nach Anspruch 1, wobei der Körper (200) aus einem medizinischen Harz oder anderem für den Menschen verträglichen Material hergestellt ist.

9. Orale Vorrichtung nach Anspruch 1, wobei die untere Zahnaussparung (300) so gebildet ist, dass die Tiefe der unteren Zahnaussparung nicht größer als eine Höhe einer freiliegenden Oberfläche der Unterkieferzähne des Patienten ist.

## Revendications

1. Appareil buccal pouvant être fixé à des dents de mandibule d'un patient comprenant:
un corps (200) ayant une forme correspondant à un ensemble de dents ;
un évidement de dents inférieures (400) formé dans une partie inférieure du corps pour permettre aux dents de mandibule d'y être insérées ;
un évidement de dents supérieures (300) formé dans une partie supérieure du corps pour permettre aux dents de maxillaire d'y être insérées et permettre à la mandibule de se déplacer vers l'arrière sur une distance prédéterminée ; et
une partie de guidage de dents supérieures (710) formée pour s'étendre du corps pour faire face à une surface latérale des dents de maxillaire,
dans lequel l'évidement de dents inférieures comprend en outre un évidement de fixation de molaires inférieures (810) formé pour venir en prise fortement avec au moins une partie des molaires gauches et droites des dents de mandibule par l'intermédiaire d'une force de frottement et un évidement d'incisives inférieures (820) formé pour permettre à d'autres dents de mandibule, à l'exception des molaires gauches et droite, de s'y positionner,
dans lequel l'évidement d'incisives inférieures (820) est formé pour être espacé des incisives inférieures, et ainsi l'évidement d'incisives inférieures ne vient pas en contact avec les incisives inférieures,
dans lequel l'évidement de dents supérieures (300) est formé pour être espacé des dents de maxillaire, et ainsi l'évidement de dents supérieures ne vient pas fortement en prise avec les dents de maxillaire,
dans lequel l'évidement de dents supérieures (300) et l'évidement de dents inférieures (400) sont formés de telle sorte que les dents de mandibule du patient avancent plus qu'avant de porter l'appareil buccal fixé aux dents de mandibule.

2. Appareil buccal selon la revendication 1, comprenant en outre une partie de montage de pointe de langue (900) formée sur une partie du corps pour permettre à la langue du patient d'être placée dessus.

3. Appareil buccal selon la revendication 1, dans lequel une limite entre l'évidement de fixation de molaires inférieures (810) et l'évidement d'incisives inférieures (820) est une canine.

4. Appareil buccal selon la revendication 1, dans lequel la partie de guidage de dents supérieures (710) est formée de telle sorte qu'une longueur étendue d'une région de molaires supérieures est plus grande qu'une longueur étendue d'une région d'incisives supérieures.

5. Appareil buccal selon la revendication 4, dans lequel la partie de guidage de dents supérieures (710) est formée pour s'étendre à partir du corps (200) vers un espace entre une joue et des dents de maxillaire du patient pour faire face à un côté extérieur des dents de maxillaire.

6. Appareil buccal selon la revendication 4, dans lequel la partie de guidage de dents supérieures (710) est formée pour s'étendre vers un palais du patient pour faire face à un côté intérieur des dents de maxillaire.

7. Appareil buccal selon la revendication 4, dans lequel la partie de guidage de dents supérieures (710) comporte :
une première partie de guidage de dents supérieures formée pour s'étendre à partir du corps vers un espace entre une joue et des dents de maxillaire du patient pour faire face à un côté extérieur des dents de maxillaire ; et
une seconde partie de guidage de dents supérieures formée pour s'étendre à partir du corps vers le palais du patient pour faire face à un côté intérieur des dents de maxillaire.

8. Appareil buccal selon la revendication 1, dans lequel le corps (200) est réalisé en une résine médicale ou en un autre matériau sans danger pour l'homme.

9. Appareil buccal selon la revendication 1, dans lequel l'évidement de dents inférieures (300) est formé de telle sorte que la profondeur de l'évidement de dents inférieures n'est pas supérieure à une hauteur d'une surface exposée des dents de mandibule du patient.
